# FASCICULE DE BREVET EUROPEEN

(11) **EP 2 151 584 B1**
(45) Date de publication et mention de la délivrance du brevet: **25.01.2012**
(21) Numéro de dépôt: 09175343.4
(22) Date de dépôt: 28.03.2001
(51) Int. Cl.: F04D 29/42, F04D 25/06, F04D 29/28

(54) **Ventilateur centrifuge**
Kreisellüfter
Centrifugal fan

(30) Priorité: 30.03.2000 FR 0004068
(43) Date de publication de la demande: 10.02.2010
(62) Demande divisionnaire de: 01400803.1
(73) Titulaire: Covidien AG, 8212 Neuhausen am Rheinfall (CH)
(72) Inventeur: Darnis, Olivier, 31150 Bruguières (FR); Fontalbat, Thierry, 31120 Portet sur Garonne (FR)
(74) Mandataire: Beyer, Andreas

(56) Documents cités:
- EP-A- 0 487 141
- WO-A-91/17361
- US-A- 2 796 836
- US-A- 2 830 755
- US-A- 5 363 674
- US-A- 5 639 217
- US-A- 5 924 847

## Description

La présente invention concerne un système d'assistance respiratoire.

Pour les malades souffrant d'insuffisance respiratoire, il est connu des machines d'assistance respiratoire permettant d'insuffler aux malades de l'air ambiant au travers d'un masque maintenu en regard des voies respiratoires du patient.

Une telle machine d'assistance respiratoire comporte un ventilateur de mise en mouvement du flux d'air. Ce flux d'air est acheminé jusqu'au masque appliqué sur le visage du patient par des tuyauteries adaptées. Un exemple d'une telle machine est fourni par GB-A-1 407 408.

Les ventilateurs utilisés dans les machines d'assistance respiratoire sont des ventilateurs de petite taille ayant un coût de fabrication réduit. Ils fonctionnent généralement à une vitesse de rotation de l'ordre de 10.000 à 20.000 tours par minute.

On constate que les machines d'assistance respiratoire émettent, en fonctionnement, un bruit important qui occasionne des nuisances sonores pour le patient.

Le bruit émis provient essentiellement du ventilateur utilisé pour mettre l'air en mouvement.

L'invention a pour but de proposer un ventilateur de taille très réduite pouvant être implanté dans une machine d'assistance respiratoire et dont les émissions sonores sont réduites.

A cet effet, l'invention a pour objet un système d'assistance respiratoire, tel que défini à la revendication 1. Dans le contexte de l'invention, l'expression "à haute vitesse" signifie "à une vitesse supérieure à 25.000 tours par minute.

Des modes particuliers de réalisation sont spécifiés aux revendications dépendantes 2 à 15.

L'invention sera mieux comprise à la lecture de la description qui va suivre, donnée uniquement à titre d'exemple et faite en se référant aux dessins, sur lesquels:
- la figure 1 est une vue en coupe longitudinale d'un ventilateur centrifuge appartenant à un dispositif selon l'invention ;

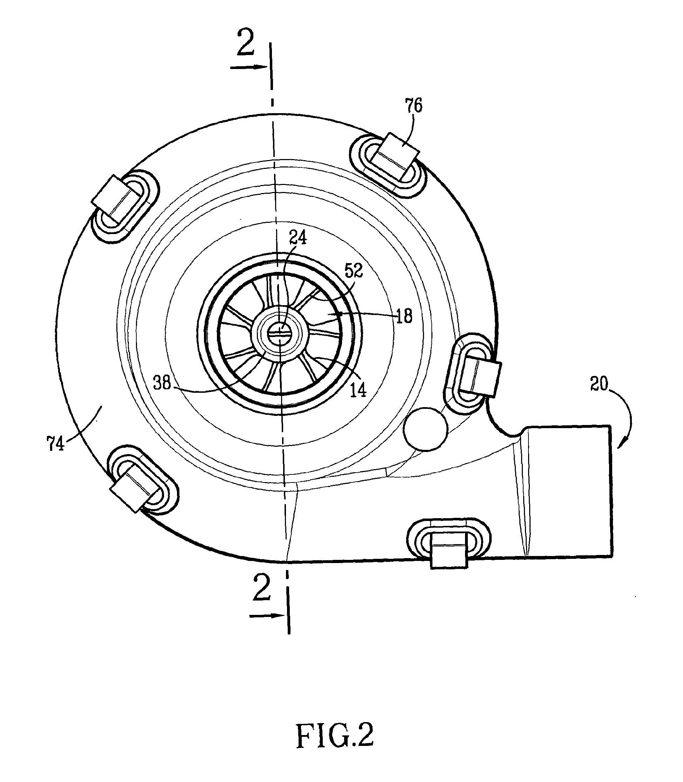

- la figure 2 est une vue en bout du ventilateur;
- la figure 3 est une vue en perspective de la roue à aubes du ventilateur des figures 1 et 2 ;
- la figure 4 est une vue en élévation de la roue à aubes de la figure 3 dont le flasque a été retiré ; et
- la figure 5 est une vue en coupe du carter du ventilateur de la figure 1 suivant son plan médian dans lequel tourne la roue à aubes.

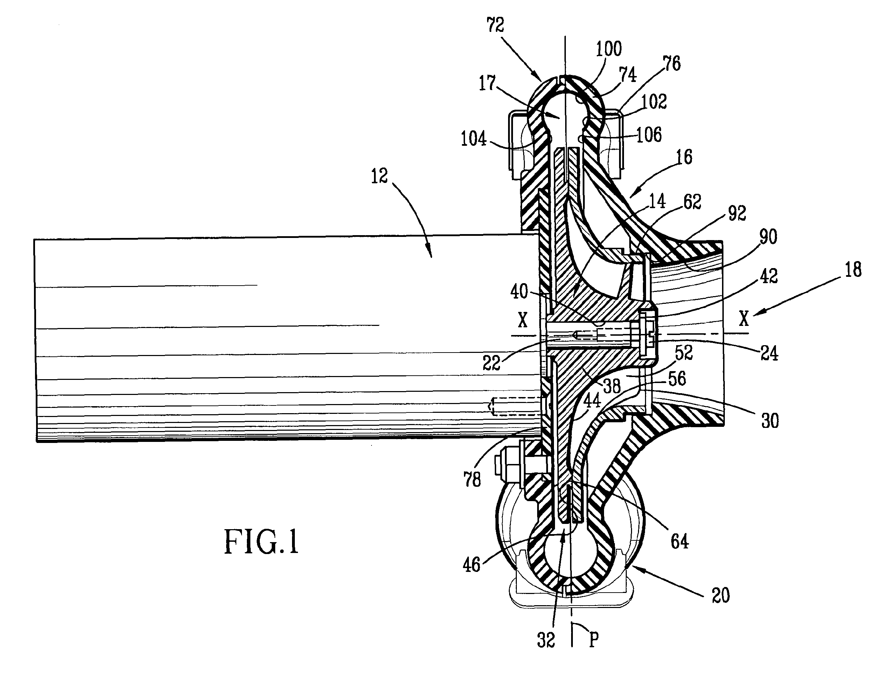

Le ventilateur représenté sur la figure 1 comporte essentiellement un moteur électrique 12, une roue à aubes 14 entraînée par le moteur électrique et un carter 16 délimitant une volute 17 dans laquelle la roue à aubes 14 est montée rotative.

Le carter 16 présente en son centre, suivant l'axe de la roue à aubes 14, une entrée d'aspiration d'air 18. Il présente également à sa périphérie une sortie de refoulement d'air 20 s'étendant tangentiellement à la roue 14.

Le moteur 12 est un moteur électrique de tout type adapté comportant un arbre de sortie 22 d'axe X-X. Le moteur est choisi pour que son arbre de sortie tourne à une vitesse supérieure à 25.000 tours par minute. Cette vitesse est de préférence comprise entre 47.000 et 56.000 tours par minute.

La roue à aubes 14 est fixée directement sur l'arbre de sortie du moteur à l'aide d'une vis 24. Elle est généralement d'axe X-X et est montée rotative autour de cet axe. Ainsi la roue est entraînée à la même vitesse que le moteur 12.

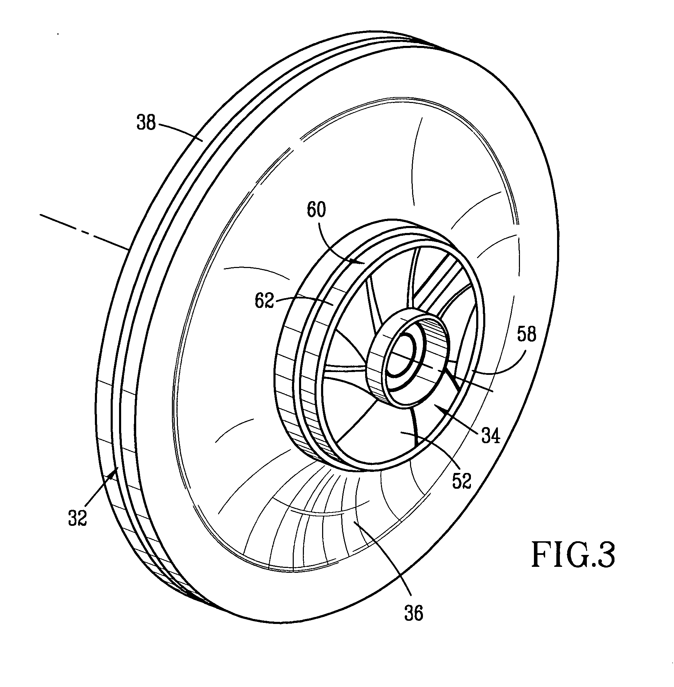

Comme illustré sur la figure 3, la roue à aubes 14 est une roue fermée comportant une bouche d'aspiration axiale 30 ouverte autour de son axe X-X de rotation et un passage périphérique 32 de refoulement centrifuge du flux gazeux ménagé à la périphérie de la roue suivant un plan P dans lequel la roue est entraînée en rotation par le moteur 12. Le passage 32 est ménagé dans l'épaisseur de la roue.

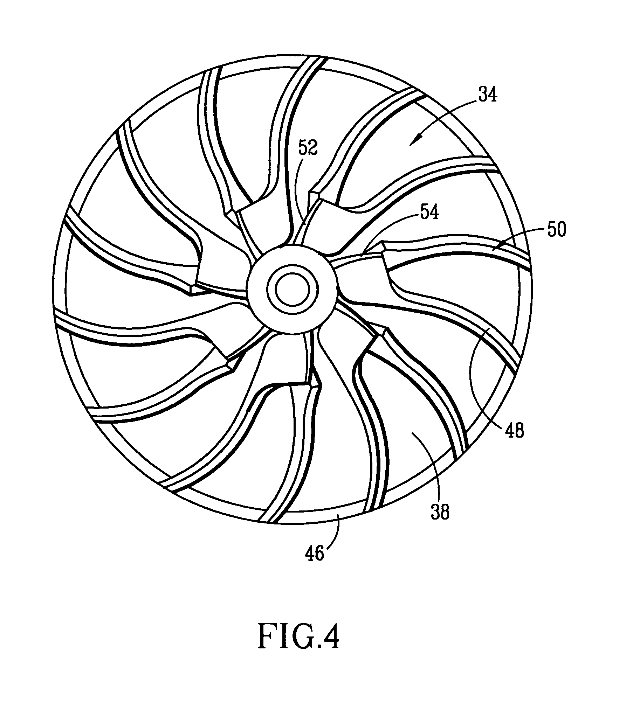

La roue à aubes 14 est formée d'un corps de roue 34 représenté seul sur la figure 4 et d'un flasque annulaire 36 rapporté coaxialement sur le corps de roue.

Le corps de roue 34 et le flasque 36 sont assemblés par exemple par collage ou ultrasons.

Le corps de roue 34 comporte un moyeu 38 traversé axialement par un alésage de réception de l'arbre 22. L'alésage présente à une extrémité un suralésage de réception d'une rondelle d'appui de la tête de vis 24 pour le montage de la roue sur l'arbre 22 du moteur.

Le moyeu 38 est de révolution d'axe X-X. Il présente un diamètre progressivement croissant depuis son extrémité avant délimitant la bouche d'aspiration 30 jusqu'à son extrémité arrière présentant une forme générale de disque et délimitant le passage périphérique de refoulement 32. Ainsi, le moyeu 34 présente une surface latérale 44 de révolution dont le profil s'évase continûment de la bouche d'aspiration 30 au passage périphérique de refoulement 32. Cette surface latérale 44 définit la veine fluide. Elle est constituée par exemple par un tronçon d'hyperboloïde de révolution d'axe X-X.

A son extrémité arrière délimitant le passage périphérique de refoulement 32, la surface 44 présente une plage annulaire plane 46 s'étendant parallèlement au plan P de sortie de la roue.

Comme illustré sur la figure 4, des aubes principales 48 et des aubes secondaires 50 font saillie à la surface latérale 44 de la roue. Ces aubes sont venues de matière avec le moyeu 38 et sont adaptées pour assurer un guidage du flux gazeux de la bouche d'aspiration 30 vers le passage périphérique de refoulement 32.

Les aubes primaires et secondaires sont de hauteur progressivement décroissante depuis la bouche d'aspiration 30 jusqu'au passage périphérique de refoulement 32, la hauteur étant mesurée suivant l'axe X-X.

Les aubes principales 48 présentent un bord d'attaque 52 s'étendant radialement dans la bouche d'aspiration 30 de la roue. Au contraire, les aubes secondaires 50 ont un bord d'attaque 54 disposé en retrait de la bouche d'aspiration 30.

Au-delà de leur bord d'attaque respectif, les profils des aubes 48 et 50 sont identiques jusqu'au passage périphérique de refoulement 32. Ces aubes définissent des surfaces gauches tri-dimensionnelles, c'est-à-dire que la normale à la surface de chaque aube varie continûment dans trois directions orthogonales suivant la longueur de chaque aube.

Le flasque 36 présente une forme extérieure et intérieure généralement tronconique. Sa surface intérieure, notée 56, est adaptée pour s'appliquer exactement sur la surface longitudinale libre des aubes primaires et secondaires. Ainsi, les aubes délimitent entre elles, entre la surface latérale 44 du moyeu et la surface intérieure 56 du flasque rapporté, des canaux indépendants de guidage du flux gazeux. Ces canaux ont une section progressivement croissante de la bouche d'aspiration 30 jusqu'au passage périphérique de refoulement 32.

Le flasque 36 présente, en son centre, un tronçon cylindrique 58 définissant, avec l'extrémité avant du moyeu, la bouche d'aspiration 30. Les bords d'attaque 52 des aubes principales sont entourés par ce tronçon cylindrique 58.

A son extrémité libre, la surface extérieure du tronçon cylindrique 58 présente une surface cylindrique 62 adaptée pour être disposée avec un jeu faible en regard d'un lamage correspondant ménagé dans le carter.

A sa périphérie extérieure, dans la région délimitant le passage périphérique de refoulement 32 de la roue, le flasque 36 présente une plage annulaire plane 64 s'étendant sensiblement parallèlement au plan de sortie P de la roue.

Ainsi, le passage périphérique de refoulement 32 est délimité par les deux plages annulaires 46 et 64 s'étendant presque parallèlement l'une à l'autre. La plage 46 est perpendiculaire à l'axe X-X, alors que la plage 64 est légèrement conique. Ces deux plages assurent le guidage du flux gazeux pour sa sortie de la roue suivant le plan P perpendiculaire à l'axe X-X de rotation.

Le carter 16 comporte deux demi-coquilles 72, 74 reliées l'une à l'autre suivant un plan médian correspondant au plan P de sortie de la roue 14. Les deux demi-coquilles 72 et 74 sont réalisées en matière plastique injectée. Elles sont reliées l'une à l'autre par des agrafes métalliques 76 réparties suivant la périphérie du carter.

La demi-coquille 72 est reliée à la partie fixe du moteur électrique 12 par une plaque 78 qui est vissée à la partie fixe du moteur et sur laquelle est boulonnée la demi-coquille 72.

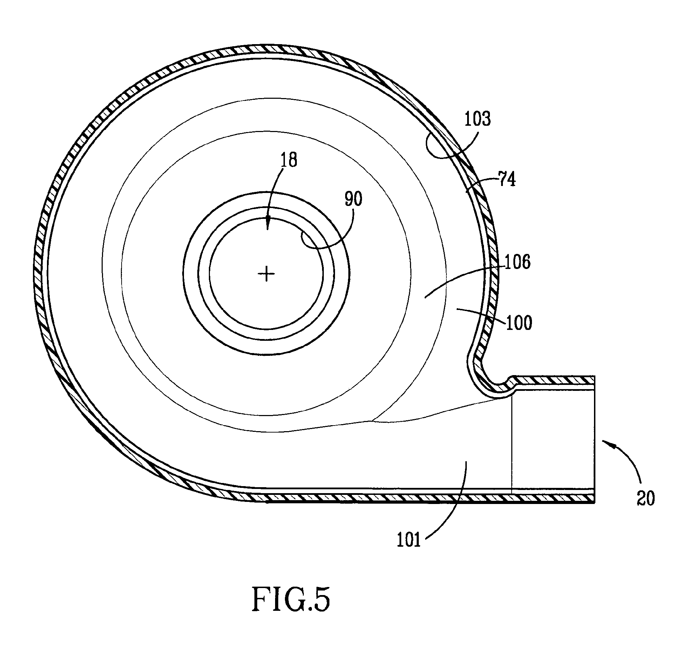

L'entrée d'aspiration 18 est définie dans la demi-coquille 74 représentée seule sur la figure 5. Cette entrée 18 est délimitée par un tronçon convergent 90 s'étendant de l'extrémité ouverte de la demi-coquille 74 jusqu'à la bouche d'aspiration 30 de la roue. En arrière du tronçon d'aspiration 90, la surface intérieure de la demi-coquille 74 comporte un lamage axial 92 qui est appliqué avec un jeu faible en regard de la surface cylindrique 62 de la roue, assurant ainsi l'étanchéité entre le tronçon convergent d'entrée 90 et la volute 17 définie par le carter.

La volute 17 est définie, entre les demi-coquilles 72 et 74. Elle comporte, à la périphérie de la roue 14, en regard du passage périphérique de refoulement 32, un canal circulaire 100 de guidage du flux gazeux.

Comme illustré sur la figure 5, le canal périphérique 100 de guidage du flux gazeux est intérieurement évolutif dans le plan de rotation de la roue. Il définit une spirale centrée sur l'axe X-X de la roue. Le canal 100 s'ouvre sur l'espace médian défini entre les deux demi-coquilles et dans lequel s'étend la roue à aubes 14.

Le canal 100 est relié à la sortie de refoulement par un conduit rectiligne 101 divergeant délimité par les deux demi-coquilles 72, 74. Ce conduit 101 s'étend suivant une direction tangentielle au canal 100. Son diamètre est progressivement croissant du canal 100 vers la sortie de refoulement 20.

Selon l'invention, et comme illustré sur la figure 1, le canal 100 est symétrique en section par rapport au plan P de sortie de la roue. Ce canal présente à sa base une région 102 sensiblement circulaire en section. Cette région sensiblement circulaire est centrée suivant un point du plan P de sortie de la roue. La section de la région sensiblement circulaire 102 augmente progressivement en direction de la sortie de refoulement 20. Elle est croissante sur l'essentiel de la périphérie du carter. Cette région 102 sensiblement circulaire en section s'enroule suivant un contour extérieur 103 circulaire centré sur l'axe X-X. Ainsi, la volute 17 a extérieurement un contour circulaire et intérieurement un contour en spirale. La région 102 s'étend en section sur une plage angulaire supérieure à 180°.

De part et d'autre, la région sensiblement circulaire 102 prévue à la base du canal est prolongée par deux surfaces planes parallèles 104, 106 ménagées respectivement sur les faces intérieures des demi-coquilles 72 et 74. Ces surfaces s'étendent parallèlement au plan P de sortie de la roue.

Le bord extérieur de la roue, dans l'épaisseur duquel est ménagé le passage périphérique de refoulement 32 s'étend en dehors de la région sensiblement circulaire 102 du canal périphérique. Ainsi, le bord extérieur s'étend entre les deux surfaces planes annulaires 104 et 106.

Le canal périphérique 100 de guidage du flux gazeux défini à la périphérie de la volute 17 présente une surface essentiellement lisse et l'espace délimité entre cette surface lisse et le passage périphérique de refoulement 32 de la roue est exempt de tout diffuseur, cet espace étant maintenu libre pour l'écoulement du flux gazeux.

Avantageusement, la roue à aubes 14 est dimensionnée, et notamment ses aubes 48 et 50, de sorte que le taux de compression du ventilateur soit compris entre 1,05 et 1,12.

Dans un tel ventilateur centrifuge, l'air est aspiré par l'entrée d'aspiration 18 et est rejeté par la sortie de refoulement 20.

La vitesse élevée de rotation de la roue 14 et la symétrie du canal périphérique 100 par rapport au plan de sortie des gaz issus de la roue permettent un fonctionnement avec des émissions sonores très réduites, tout en permettant d'assurer un débit satisfaisant de l'ordre de 2,5 I/s avec une élévation de la pression à 70 mbars. Pour un tel débit, le niveau de pression sonore à l'aspiration est de 64 dBA.

De plus, les caractéristiques du ventilateur permettent d'obtenir un fonctionnement stable de celui-ci sur toute la plage de débits.

Un tel ventilateur centrifuge convient ainsi parfaitement pour un dispositif d'assistance respiratoire.

Dans un tel dispositif, le ventilateur est monté dans un bâti. La sortie du ventilateur est reliée à une extrémité d'un flexible, l'autre extrémité du flexible étant adaptée pour recevoir un masque de ventilation.

Un dispositif d'assistance respiratoire équipé d'un tel ventilateur ne crée que de faibles nuisances sonores, améliorant ainsi le confort de l'utilisateur.

## Revendications

1. Dispositif d'assistance respiratoire pour fournir une assistance respiratoire à un patient, comprenant un ventilateur centrifuge, **caractérisé en ce que** le ventilateur comporte un carter (16) définissant intérieurement une volute (17) de guidage du gaz, une roue à aubes (14) d'entraînement du gaz montée rotative dans la volute (17), et des moyens (12) d'entraînement à haute vitesse de la roue à aubes (14), la roue à aubes présentant, autour de son axe (X-X), une bouche (30) d'aspiration du gaz et, à sa périphérie, un passage (32) de refoulement du gaz suivant un plan (P) de sortie de la roue (14), le carter (16) présentant en outre une sortie (20) de refoulement du gaz en dehors de la volute (17), et la volute (17) définissant, à sa périphérie, un canal (100) de guidage du gaz qui est essentiellement symétrique en section par rapport au plan (P) de sortie de ladite roue.

2. Dispositif d'assistance respiratoire selon la revendication 1, **caractérisé en ce que** la roue comporte, des aubes (48, 50) de part et d'autre desquelles sont ménagées des parois pleines (36, 38) annulaires, les parois pleines annulaires et les aubes définissant des conduits disjoints de guidage du gaz.

3. Dispositif d'assistance respiratoire selon la revendication 1 ou 2, **caractérisé en ce que** chaque aube (48, 50) définit une surface gauche dont la normale varie suivant trois directions orthogonales le long de la longueur de l'aube.

4. Dispositif d'assistance respiratoire selon l'une quelconque des revendications précédentes, **caractérisé en ce que** le taux de compression est compris entre 1,05 et 1,12.

5. Dispositif d'assistance respiratoire selon l'une quelconque des revendications précédentes, **caractérisé en ce que** la section du canal périphérique (100) de guidage du flux gazeux défini à la périphérie de la volute (17) est progressivement croissante en direction de la sortie (20) de refoulement du gaz en dehors de la volute (17).

6. Dispositif d'assistance respiratoire selon l'une quelconque des revendications précédentes, **caractérisé en ce que** le canal périphérique (100) de guidage du gaz défini à la périphérie de la volute (17) décrit extérieurement un contour essentiellement circulaire (103) dans le plan de sortie de la roue (14), lequel contour circulaire (103) est centré sur l'axe (X-X) de rotation de la roue.

7. Dispositif d'assistance respiratoire selon l'une quelconque des revendications précédentes, **caractérisé en ce que** le canal périphérique (100) de guidage du gaz défini à la périphérie de la volute (17) comporte, à sa base, une région (102) sensiblement circulaire en section et, symétrique par rapport au plan P de sortie de la roue.

8. Dispositif d'assistance respiratoire selon la revendication 7, **caractérisé en ce que** la région (102) sensiblement circulaire en section s'étend sur une plage angulaire supérieure à 180°.

9. Dispositif d'assistance respiratoire selon la revendication 6 ou 7, **caractérisé en ce que** le bord périphérique de la roue s'étend en dehors de la région (102) sensiblement circulaire en section définie à la base du canal périphérique de guidage du gaz.

10. Dispositif d'assistance respiratoire selon l'une quelconque des revendications précédentes, **caractérisé en ce que** l'espace entre le canal périphérique (100) de guidage du gaz et le passage périphérique de refoulement du gaz (32) est dépourvu de tout obstacle pour l'écoulement libre du gaz.

11. Dispositif d'assistance respiratoire selon l'une quelconque des revendications précédentes, **caractérisé en ce que** les moyens (12) sont adaptés pour entraîner la roue à aubes (14) à une vitesse supérieure à 25.000 tours par minute.

12. Dispositif d'assistance respiratoire selon la revendication 11, **caractérisé en ce que** ladite vitesse est comprise entre 47.000 et 56.000 tours par minute.

13. Dispositif d'assistance respiratoire selon l'une quelconque des revendications précédentes, **caractérisé en ce que** la bouche (30) s'étend en regard d'une entrée d'aspiration (18) ménagée dans le carter (16).

14. Dispositif d'assistance respiratoire selon l'une quelconque des revendications précédentes, **caractérisé en ce que** le passage (32) est adapté pour refouler le gaz suivant le plan de sortie (P) de la roue (14) de manière centrifuge.

15. Dispositif d'assistance respiratoire selon l'une quelconque des revendications précédentes, **caractérisé en ce que** le dispositif comporte en outre un bâti, dans lequel est monté le ventilateur, et un flexible, dont une extrémité est reliée à la sortie (20) du carter (16) et dont l'autre extrémité est adaptée pour recevoir un masque de ventilation.

## Claims

1. A respiratory assistance device for supplying respiratory assistance to a patient, comprising a centrifugal fan, **characterised in that** it comprises a casing (16) defining an inner gas-guiding volute (17), a rotating gas-driving vane wheel (14) mounted inside the volute (17), and means (12) for driving the vane wheel (14) at high speed, the vane wheel having, around its axis (X-X), a gas-suction opening (30) and, on its outer part, a passage (32) for discharging the gas following an outlet plane (P) of the wheel (14), the casing (16) in addition having an outlet (20) for discharging the gas out of the volute (17), and the volute (17) defining, on its outer part, a gas-guiding duct (100) whose section is essentially symmetrical in relation to the outlet plane (P) of said wheel.

2. A respiratory assistance device according to claim 1, **characterised in that** the wheel comprises vanes (48, 50) around which are arranged solid annular walls (36, 38), the solid annular walls and the vanes defining independent gas-guiding ducts.

3. A respiratory assistance device according to claims 1 or 2, **characterised in that** each vane (48, 50) defines a warped surface, of which the normal will vary in accordance with three orthogonal directions along the whole length of the vane.

4. A respiratory assistance device according to any one of the previous claims, **characterised in that** the compression ratio is comprised between 1.05 and 1.12.

5. A respiratory assistance device according to any one of the previous claims, **characterised in that** the section of the peripheral gas-guiding duct (100), defined on the outer part of the volute (17), increases progressively in the direction of the outlet (20) for discharging the gas outside of the volute (17).

6. A respiratory assistance device according to any one of the previous claims, **characterised in that** the peripheral gas-guiding duct (100), defined on the outer part of the volute (17), describes an essentially circular external outline (103) within the outlet plane of the wheel (14), said circular outline (103) being centred on the rotating axis (X-X) of the wheel.

7. A respiratory assistance device according to any one of the previous claims, **characterised in that** the peripheral gas-guiding duct (100), defined on the outer part of the volute (17), comprises, at its base, a substantially circular sectional region (102), which is symmetrical in relation to the outlet plane (P) of the wheel.

8. A respiratory assistance device according to claims 7, **characterised in that** the substantially circular sectional region (102) extends along an angular range exceeding 180°.

9. A respiratory assistance device according to claims 6 or 7, **characterised in that** the peripheral edge of the wheel extends outside the substantially circular sectional region (102), defined at the base of the gas-guiding peripheral duct.

10. A respiratory assistance device according to any one of the previous claims, **characterised in that** the space between the gas-guiding peripheral duct (100) and the peripheral passage (32) for discharging the gas encounters no hindrance for the free flow of the gas.

11. A respiratory assistance device according to any one of the previous claims, **characterised in that** the means (12) are adapted in order to drive the vane wheel (14) at a speed exceeding 25,000 revolutions per minute.

12. A respiratory assistance device according to claim 11, **characterised in that** said speed is comprised between 47,000 and 56,000 revolutions per minute.

13. A respiratory assistance device according to any one of the previous claims, **characterised in that** the opening (30) extends in order to face a suction inlet (18) arranged inside the casing (16).

14. A respiratory assistance device according to any one of the previous claims, **characterised in that** the passage (32) is adapted in order to centrifugally discharge the gas following the outlet plan of the wheel (14).

15. A respiratory assistance device according to any one of the previous claims, **characterised in that** the device also comprises a structure, inside which is mounted the fan, and a tube, one tip of which being connected to the outlet (20) of the casing (16), and the other tip of which being adapted in order to fit a ventilation mask.

## Patentansprüche

1. Atmungsunterstützende Vorrichtung zur Unterstützung der Atmung eines Patienten, mit einem Zentrifugalgebläse,
**dadurch gekennzeichnet, dass** das Gebläse ein Gehäuse (16), das im Inneren ein Spiralgehäuse (17) zum Leiten des Gases bildet, ein im Spiralgehäuse (17) drehbar angebrachtes Laufrad (14) zum Befördern des Gases und Mittel (12) zum Hochgeschwindigkeitsantrieb des Laufrades (14) umfasst, wobei das Laufrad um seine Achse (X-X) eine Öffnung (30) zum Ansaugen des Gases und an seiner Außenfläche einen Durchlass (32) zur Förderung des Gases entlang einer Austrittsebene (P) des Rades (14) aufweist, wobei das Gehäuse (16) ferner einen Auslass (20) zur Förderung des Gases aus dem Spiralgehäuse (17) hinaus aufweist, und das Spiralgehäuse (17) an seiner Außenfläche einen Gasführungskanal (100) bildet, der bezüglich der Austrittsebene (P) des Rades im Querschnitt im Wesentlichen symmetrisch ist.

2. Atmungsunterstützende Vorrichtung nach Anspruch 1,
**dadurch gekennzeichnet, dass** das Rad Schaufeln (48, 50) aufweist, zu deren beiden Seiten massive Ringwände (36, 38) ausgebildet sind, wobei die massiven Ringwände und die Schaufeln voneinander getrennte Gasführungskanäle bilden.

3. Atmungsunterstützende Vorrichtung nach Anspruch 1 oder 2,
**dadurch gekennzeichnet, dass** jede Schaufel (48, 50) eine verwundene Fläche bildet, deren Normale sich in drei orthogonalen Richtungen entlang der Schaufel ändert.

4. Atmungsunterstützende Vorrichtung nach einem der vorhergehenden Ansprüche,
**dadurch gekennzeichnet, dass** das Verdichtungsverhältnis zwischen 1,05 und 1,12 beträgt.

5. Atmungsunterstützende Vorrichtung nach einem der vorhergehenden Ansprüche,
**dadurch gekennzeichnet, dass** der Querschnitt des an der Außenfläche des Spiralgehäuses (17) ausgebildeten peripheren Gasführungskanals (100) allmählich in Richtung des Auslasses (20) zur Förderung des Gases aus dem Spiralgehäuse (17) hinaus zunimmt.

6. Atmungsunterstützende Vorrichtung nach einem der vorhergehenden Ansprüche,
**dadurch gekennzeichnet, dass** der an der Außenfläche des Spiralgehäuses (17) ausgebildete periphere Gasführungskanal (100) außen eine im Wesentlichen kreisförmige Umrisslinie (103) in der Austrittsebene des Rades (14) beschreibt, wobei die kreisförmige Umrisslinie (103) auf die Drehachse (X-X) des Rades zentriert ist.

7. Atmungsunterstützende Vorrichtung nach einem der vorhergehenden Ansprüche,
**dadurch gekennzeichnet, dass** der an der Außenfläche des Spiralgehäuses (17) ausgebildete periphere Gasführungskanal (100) an seiner Basis einen im Querschnitt im Wesentlichen kreisförmigen und bezüglich der Austrittsebene (P) des Rades symmetrischen Bereich (102) aufweist.

8. Atmungsunterstützende Vorrichtung nach Anspruch 7,
**dadurch gekennzeichnet, dass** sich der im Querschnitt im Wesentlichen kreisförmige Bereich (102) über einen Winkelbereich von mehr als 180° erstreckt.

9. Atmungsunterstützende Vorrichtung nach Anspruch 6 oder 7,
**dadurch gekennzeichnet, dass** der periphere Rand des Rades außerhalb des im Querschnitt im Wesentlichen kreisförmigen, an der Basis des peripheren Gasführungskanals ausgebildeten Bereichs (102) verläuft.

10. Atmungsunterstützende Vorrichtung nach einem der vorhergehenden Ansprüche,
**dadurch gekennzeichnet, dass** der Raum zwischen dem peripheren Gasführungskanal (100) und dem peripheren Gasförderdurchlass (32) für ein ungehindertes Strömen des Gases frei von jeglichem Hindernis ist.

11. Atmungsunterstützende Vorrichtung nach einem der vorhergehenden Ansprüche,
**dadurch gekennzeichnet, dass** die Mittel (12) das Laufrad (14) mit einer Geschwindigkeit von über 25.000 Umdrehungen pro Minute anzutreiben vermögen.

12. Atmungsunterstützende Vorrichtung nach Anspruch 11,
**dadurch gekennzeichnet, dass** die Geschwindigkeit zwischen 47.000 und 56.000 Umdrehungen pro Minute beträgt.

13. Atmungsunterstützende Vorrichtung nach einem der vorhergehenden Ansprüche,
**dadurch gekennzeichnet, dass** sich die Öffnung (30) gegenüber einem im Gehäuse (16) ausgebildeten Saugeinlass (18) erstreckt.

14. Atmungsunterstützende Vorrichtung nach einem der vorhergehenden Ansprüche,
**dadurch gekennzeichnet, dass** der Durchlass (32) das Gas entlang der Austrittsebene (P) des Rades (14) zentrifugal zu fördern vermag.

15. Atmungsunterstützende Vorrichtung nach einem der vorhergehenden Ansprüche,
**dadurch gekennzeichnet, dass** die Vorrichtung ferner ein Gestell, in dem das Gebläse montiert ist, und einen Schlauch umfasst, von dem ein Ende mit dem Auslass (20) des Gehäuses (16) verbunden ist und das andere Ende eine Beatmungsmaske aufzunehmen vermag.
